# EUROPEAN PATENT SPECIFICATION

(11) **EP 2 568 982 B1**
(45) Date of publication and mention of the grant of the patent: **02.04.2014**
(21) Application number: 11718734.4
(22) Date of filing: 09.05.2011
(51) Int. Cl.: A61K 31/426, A61P 9/12, A61K 31/41, A61K 31/4178, A61K 31/4184

(54) **ASSOCIATION OF XANTHINE OXIDASE INHIBITORS AND ANGIOTENSIN II RECEPTOR ANTAGONISTS AND USE THEREOF**
ASSOZIATION VON XANTHINOXIDASEHEMMERN UND ANGIOTENSIN II REZEPTORANTAGONISTEN UND IHRE VERWENDUNG
ASSOCIATION D'INHIBITEURS DE LA XANTHINE OXYDASE ET D'ANTAGONISTES DU RÉCEPTEUR DE L'ANGIOTENSINE II ET SON UTILISATION

(30) Priority: 10.05.2010 IT RM20100232
(43) Date of publication of application: 20.03.2013
(73) Proprietor: Menarini International Operations Luxembourg S.A., 1611 Luxembourg (LU)
(72) Inventor: MELANI, Francesco, I-50014 Fiesole FI (IT); GIULIANI, Sandro, I-50512 Bagno A Ripoli FI (IT); MAGGI, Carlo Alberto, I-50125 Firenze FI (IT)
(74) Representative: Germinario, Claudio
(86) International application number: PCT/EP2011/057438
(87) International publication number: WO 2011/141431

(56) References cited:
- WO-A2-2007/019153
- TAKAHASHI S ET AL: "Effects of combination treatment using anti-hyperuricaemic agents with fenofibrate and/or losartan on uric acid metabolism", ANNALS OF THE RHEUMATIC DISEASES, vol. 62, no. 6, 1 June 2003 (2003-06-01), pages 572-575, XP008127491, BRITISH MEDICAL ASSOCIATION, LONDON, GB ISSN: 0003-4967
- SORBERA L A ET AL: "TMX-67: Treatment of gout and hyperuricemia, xanthine oxidase inhibitor: TEI-6720", DRUGS OF THE FUTURE, vol. 26, no. 1, 1 January 2001 (2001-01-01), pages 32-38, XP002553641, PROUS SCIENCE, ES ISSN: 0377-8282
- BOMALASKI JOHN S ET AL: "Serum uric acid-lowering therapies: where are we heading in management of hyperuricemia and the potential role of uricase.", CURRENT RHEUMATOLOGY REPORTS, vol. 6, no. 3, June 2004 (2004-06), pages 240-247, XP002603882, ISSN: 1523-3774
- LEE S J ET AL: "New developments in clinically relevant mechanisms and treatment of hyperuricemia", CURRENT RHEUMATOLOGY REPORTS, vol. 8, no. 3, June 2006 (2006-06), pages 224-230, XP002604269, US
- WORTMANN R L: "Recent advances in the management of gout and hyperuricemia", CURRENT OPINION IN RHEUMATOLOGY, vol. 17, no. 3, 1 January 2005 (2005-01-01), pages 319-324, XP009125112, CURRENT SCIENCE, LONDON, GB ISSN: 1040-8711, DOI: 10.1097/01.BOR.0000162060.25895.A5
- GOODMAN G A ET AL: "GOODMAN and GILMAN'S The Pharmacological Basis of Therapeutics , (Angiotensin II receptor antagonists)", 1 January 2001 (2001-01-01), GOODMAN AND GILMAN'S THE PHARMACOLOGICAL BASIS OF THERAPEUTICS, NEW YORK : MCGRAW-HILL, US, XP002604270, ISBN: 978-0-07-135469-1 page 894, column 1, paragraph 2 - page 895, column 1, paragraph 2

## Description

### FIELD OF THE INVENTION

The present invention relates to an association of the xanthine oxidase inhibitor, febuxostat, with the angiotensin II receptor antagonists, olmesartan, pharmaceutical compositions comprising said active principles, for use in a therapeutic treatment in humans or animals, and methods for the preparation thereof.

Such associations and such compositions proved particularly effective in the treatment of hypertension, alone or in association with hyperuricemia and/or hyperglycemia or to other disorders in the clinical context of the metabolic syndrome.

### STATE OF THE PRIOR ART

Gout is an invalidating chronic disease characterized by hyperuricemia and deposition of monosodium urate crystals in various tissues, mainly at the joint level and in the kidney. Hyperuricemia and gout are frequently associated to other cardiovascular risk factors such as hypertension and other elements that are part of the metabolic syndrome, like obesity, fasting hyperglycemia, low HDL levels and high triglyceride levels.

Hence, the need to always have novel means of treatment in order to better manage chronic therapy of gout and pathologies frequently correlated thereto.

A xanthine oxidase inhibitor well-known in the literature is allopurinol. More recently, other xanthine oxidase inhibitors have appeared on the market; among them, febuxostat is of particular relevance.

Febuxostat is a powerful non-purine selective inhibitor of xanthine oxidase which in clinical studies has been shown to reduce hyperuricemia more effectively than allopurinol.

Febuxostat is a thiazole derivative having formula (I), belonging to the class of xanthine oxidase inhibitors, and was originally described in EP513379.

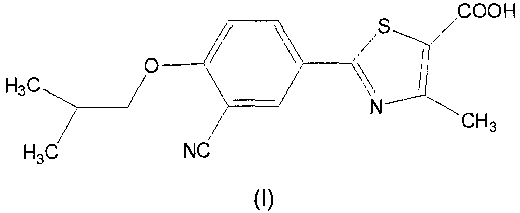

In EP1020454 it is also described a polymorphic form of febuxostat and a process for obtaining it.

In addition to its use as anti-hyperuricemic agent and in the treatment of gout, references are also found to the potential use of febuxostat in other pathologies.

In WO2004060489 it is described the use of xanthine oxidase inhibitors for increasing cardiac contractility in CHF (Chronic Heart Failure) patients.

In WO2007062028 febuxostat is used to reduce the QT interval in patients in which such interval is prolonged, and in the pathologies associated thereto.

In WO2008064015 the use of xanthine oxidase, among which febuxostat, is indicated to preserve renal function.

In W02007019153 it is claimed the use of some xanthine oxidase inhibitors, among which febuxostat, preferably for the treatment of prehypertension characterized by systolic pressure between 120 and 139 mmHg and diastolic pressure between 80 and 89 mmHg; here, xanthine oxidase inhibitors seem to be indicated also in the treatment of more marked hypertensions, though results obtained do not seem to be equal to those of already known anti-hypertensive agents.

In WO2007019153, besides the above-mentioned use it is also mentioned the optional possibility of administering to a hypertensive subject an amount of at least one anti-hypertensive compound with at least one inhibitor of a xanthine oxidase; no example however is reported with associations of a xanthine oxidase inhibitor and an anti-hypertensive agent, nor is it indicated a way of selecting, among the various classes of anti-hypertensive agents or the very large class of compounds exhibiting evident anti-hypertensive activity, that class or those compounds which may be useful for a pharmaceutical composition suitable for a treatment of hypertension combined with an anti-hypertensive agent and a xanthine oxidase inhibitor.

From the literature (Feig DL et al on JAMA 2008; 300: 924,) it is reported that allopurinol in monotherapy has shown an anti-hypertensive effect in 30 hypertensive subjects, yet only on teenagers.

Arterial hypertension is successfully treated with several drugs belonging to different therapeutic classes. Among them, the class of angiotensin II receptor antagonists must be considered of particular relevance; compounds known as 'sartans', commonly used in clinical practice and represented by the compounds selected from the group of: candesartan, eprosartan, irbesartan, losartan, olmesartan, telmisartan and valsartan.

Sartans act by blocking the angiotensin II AT1 receptor, a peptide controlling vascular tone and sodium reabsorption, i.e., blocking the hypertensive effect of angiotensin II.

In EP503785 the product olmesartan medoxil is claimed, effective in the treatment of hypertension.

In EP1336407, EP1604664, associations between olmesartan medoxil and hydrochlorotiazide or amlodipine are claimed.

### SUMMARY OF THE INVENTION

The present invention is based on the surprising discovery made by the Inventors that the association of the non-purine xanthine oxidase inhibitor, febuxostat, or pharmaceutically acceptable salts thereof or polymorphic forms thereof in combination with the angiotensin II receptor antagonist, olmesartan medoxomil or pharmaceutically acceptable salts thereof exhibits a synergistic therapeutic effect in the treatment of hypertension. In fact, experimental data reported in the present description demonstrate that the therapeutic effect resulting from the association of the two active principles is greater than the sum of the therapeutic effects resulting from the same dosages of each active principle administered alone.

A first object of the present invention is an association of the active principles:
a) the xanthine oxidase inhibitor, febuxostat, or pharmaceutically acceptable salts thereof or polymorphic forms thereof; and
b) one or more angiotensin II receptor antagonists or pharmaceutically acceptable salts thereof

A second object of the present invention is a pharmaceutical composition comprising, as active principle, a mixture of:
a) xanthine oxidase inhibitor, febuxostat, or pharmaceutically acceptable salts thereof or polymorphic forms thereof;
b) one or more angiotensin II receptor antagonists or pharmaceutically acceptable salts thereof, and
one or more carriers and/or diluents and/or pharmaceutically acceptable excipients, for use in a human or veterinary therapeutic treatment.

Another object of the present invention is a method for the preparation of the composition according to the present description, wherein the active mixture comprising:
a) the xanthine oxidase inhibitor, febuxostat, or pharmaceutically acceptable salts thereof or polymorphic forms thereof;
b) one or more angiotensin II receptor antagonists or pharmaceutically acceptable salts thereof
is formulated in suitable dosage units with one or more carriers and/or diluents and/or pharmaceutically acceptable excipients.

As an advantage with respect to the state of the prior art, the present invention advantageously entails a greater anti-hypertensive activity compared to that observed by using the sole angiotensin II receptor antagonists or the sole xanthine oxidase inhibitor. Moreover, a further advantage is given by the possibility of obtaining significant effects in the treatment of hypertension with a reduced amount of angiotensin II receptor antagonist with respect to the monotherapy treatment.

### DETAILED DESCRIPTION

The present invention relates to an association of the active principles:
a) xanthine oxidase inhibitor, febuxostat, or pharmaceutically acceptable salts thereof or polymorphic forms thereof; and
b) the angiotensin II receptor antagonist, olmesartan medoxomil or pharmaceutically acceptable salts thereof
for use in a human or veterinary therapeutic treatment.

By "association" in the present description it is meant an association of the active principles, both in the form of a physical mixture constituted by said active principles in a single dosage unit and in the form of dosage units physically separated for each active principle, but intended for a concomitant administration. In both cases, association must ensure a synergy of the therapeutic effects obtained from the individual active principles with respect to the effect obtained in monotherapy.

According to the present invention the non-purine xanthine oxidase inhibitor of said association is febuxostat, a thiazole derivative having formula (I), or pharmaceutically acceptable salts thereof or polymorphic forms thereof.

Pharmaceutically acceptable salts of febuxostat, include but are not limited to cations of alkali metals and of alkaline earth metals, such as lithium, sodium, potassium, calcium, magnesium or aluminium salts, or non-toxic derivatives with quaternary ammonium and cations of amines such as ammonium, tetramethylammonium, tetraethylammonium, methylammonium, dimethylammonium, trimethylammonium, or derive from the addition of organic amines such as ethylendiamine, ethanolamine, diethanolamine, piperazine, tromethamine, lysine, arginine and the like.

Febuxostat, its salts or polymorphic forms thereof could be obtained or prepared according to methods described in the known art, like e.g. in EP513379.

Polymorphic forms of febuxostat include, but are not limited to the forms described in European Patent EP1020454.

To the ends of the present description, the angiotensin II receptor antagonist may be chiral or non- chiral, or specific polymorphic forms thereof. In case of chiral molecules a single enantiomer, a mixture of enantiomers or diastereoisomers or the racemic mixture could be used. According to the present description those specific stereoisomers, as well as polymorphic forms, which exhibit a greater biological activity are to be preferred.

Pharmaceutically acceptable salts of the angiotensin II receptor antagonist having an acid function in the molecule include but are not limited to cations of alkali metals and of alkaline earth metals, such as lithium, sodium, potassium, calcium, magnesium or aluminium salts, or non-toxic derivatives with quaternary ammonium and cations of amines such as ammonium, tetramethylammonium, tetraethylammonium, methylammonium, dimethylammonium, trimethylammonium, or derive from the addition of organic amines such as ethylendiamine, ethanolamine, diethanolamine, piperazine, tromethamine, lysine, arginine and the like.

In a preferred embodiment said angiotensin II receptor antagonist is olmesartan medoximil or a pharmaceutically acceptable salt thereof.

In the association of the invention, the xanthine oxidase inhibitor, febuxostat, or pharmaceutically acceptable salts thereof or polymorphic forms thereof are associated with the angiotensin II receptor antagonist or pharmaceutically acceptable salts thereof in a weight ratio of febuxostat/angiotensin II receptor antagonists comprised between 0.1 and 200, or better between 0.6 and 10.

E.g., the following amounts, expressed in grams per single dose, could be associated: febuxostat in an amount comprised between 10-200 mg, or better comprised between 25-120 mg, in association with an amount of angiotensin II receptor antagonists comprised between 1-100 mg, e.g. comprised between 10-40 mg.

Where the association envisages a physical mixture of two compounds, as active principles, having the one an acid function and the other one a basic function, also the forming of an internal salt between the two is possible, in proportion to the respective amounts present in the mixture.

A further embodiment of the present invention relates to pharmaceutical compositions comprising, as active principle, a mixture of:
a) xanthine oxidase inhibitor, febuxostat, or pharmaceutically acceptable salts thereof or polymorphic forms thereof;
b) the angiotensin II receptor antagonist, olmesartan, or pharmaceutically acceptable salts thereof, and one or more usual pharmaceutically acceptable excipients, additives and/or diluents, for use in a human or veterinary therapeutic treatment.

The pharmaceutical compositions according to the present invention may be formulated in various forms depending on the selected administration route. Oral administration of solid forms my include forms such as capsules, tablets, pills, powders and granules. In these solid forms the two active principles, the xanthine oxidase inhibitor and the anti-hypertensive agent, can be mixed with one or more pharmaceutically acceptable inert excipients. Such excipients may be selected among those commonly known in the state of the art and include, but are not limited to: a) carriers, such as sodium citrate and calcium phosphate, b) fillers, such as starch, lactose, microcrystalline cellulose, sucrose, glucose, mannitol and colloidal silica, c) moistening agents, such as glycerol, d) disintegrating agents, such as alginates, calcium carbonate, starches, derivatives of starch, of cellulose and polyvinylpyrrolidone, silicates and sodium carbonate, e) binders, such as carboxymethyl cellulose, alginates, gelatin, polyvinylpyrrolidone, sucrose, polymeric derivatives of cellulose, starch derivatives, f) retarding agents, such as paraffin, cellulose polymers, fatty acid esters, g) absorption accelerators, such as quaternary ammonium compounds, h) wetting agents and surfactants, such as cetyl alcohol and glycerol monostearate, i) adsorbents, such as bentonite clays and kaolin, k) lubricants, such as talc, calcium stearate, magnesium stearate, polyethylene glycol, sodium lauryl sulfate, sodium stearyl fumarate, j) glidants, such as talc, colloidal silica.

In case the selected compositions constitute the filling of gelatin capsules, the excipients include but are not limited to compounds of the type: lactose, high molecular weight polyethylene glycol, and the like.

Solid-dosage forms may be coated with enteric, gastric coatings, or coatings of other type well-known in the state of the art. They may contain matting agents and may be of the type such as to allow the release of active ingredients only or preferably in a certain section of the intestine, optionally in a delayed manner. Substances capable of allowing such a delayed use include, but are not limited to polymers and waxes.

Liquid forms suitable for oral administration are emulsions, solutions, prepared or extemporary suspensions, syrups and elixirs. Excipients suitable for the formulations according to the present invention in liquid forms for oral use include, but are not limited to diluents commonly used in the art, such as water or other solvents, solubilizing and emulsifying agents selected from ethyl alcohol, polyalcohols, propylene glycol, glycerol, polyethylene glycol and sorbitan esters. These formulations can also contain sweeteners and aromas selected from those well-known in the state of the art.

Compositions suitable for pharmaceutically acceptable parenteral injections may comprise sterile aqueous solutions, sterile dispersions, suspensions or emulsions or powders for a reconstitution in injectable solutions or dispersions; examples of excipients suitable therefor include, but are not limited to aqueous or non-aqueous carriers, diluents, solvents or vehicles selected from: water, ethanol, polyoils (propylene or polyethylene glycol, glycerol, and the like), polyalcohols, isopropyl alcohol, ethyl acetate, benzyl alcohol, benzyl benzoate, propylene glycol, 1.3-butylene glycol, dimethylformamide, vegetable oils (in particular of olive, cotton, peanut, corn, wheat germ, olive, castor, sesame), organic esters such as ethyl oleate or the like.

These compositions may also contain preservatives of antibacterial or antifungal type, selected, yet not exclusively, from: paraben, chlorbutanol, phenol, sorbic acid and the like. It may also be useful to include an isotonic agent, e.g., a sugar, sodium chloride or the like. Moreover, pharmaceutical forms with a delayed absorption may be obtained with agents such as, for instance, yet not exclusively, aluminium monostearate and gelatin.

The suspensions, beside the active principles (xanthine oxidase inhibitors and anti-hypertensive agents), may contain suspending agents such as, for instance, yet not exclusively, ethoxylated isostearic alcohols, polyethylene sorbitol and sorbitan esters, microcrystalline cellulose, aluminium hydroxide, bentonite, alginates and cellulose derivatives in general or the like.

The right fluidity can be maintained with a coating material such as lecithin, with the maintaining of the right particle sizes in the dispersions or with the use of surfactants.

Also slow-release formulations can be prepared, by the techniques and products well-known in the state of the art.

The associations and compositions of the present invention are extremely effective in the treatment, also meant as prophylaxis and therapy, of hypertension, in humans or animals.

To the ends of the present invention it is defined "hypertension" that with diastolic values higher than ad 80 mm/Hg and/or systolic values higher than 120 mm/Hg.

Hypertension can be associated or not associated to other pathologies or symptoms. In particular, the mixtures and compositions described herein are useful also in the therapeutic treatment of hypertension associated to hyperuricemia.

Symptoms such as hypertension and hyperuricemia can also be associated to specific pathologies like, e.g., the metabolic syndrome.

By "metabolic syndrome" it is meant a clinical condition accompanied by manifestations such as obesity.

The association and compositions described herein can therefore be used in the therapeutic treatment of hypertension associated to hyperuricemia and/or other disorders in the context of the metabolic syndrome.

Dosage may vary depending on the patient's age and general conditions, the nature and seriousness of the pathology or disorder and of the administration route and type. Dosage should therefore take into account the specific condition to be treated (e.g., hyperglycemia alone or in association with hyperuricemia), the severity of the condition to be treated, the age, weight and general physical conditions of the specific patient, as well as other drugs that the patient is taking, as is well-known to those skilled in the art. Moreover, it is evident that said effective amount may, when required, be lowered or raised according to the responses of the treated patient and/or according to the assessment of the physician prescribing the compounds of the present invention.

Typically, compositions preferably for oral use in solid form contain an amount of xanthine oxidase inhibitor, specifically febuxostat, of between 10 and 200 mg per dosage unit, and preferably of from 25 to 100 mg, and an amount of angiotensin II receptor antagonist, preferably olmesartan medoximil, of between 1 and 100 or of between 10 and 40 mg per dosage unit.

By the term "dosage unit" in the present description it is meant the unitary formulation for a single administration, e.g. a tablet, capsule, etc.

By "unit dosage" it is meant the amount of active principle for a single administration.

The pharmaceutical mixtures and compositions of the invention could be prepared according to techniques known in the field both using the previously prepared association of active principles, and mixing the individual compounds directly during the preparation of the composition.

In particular, the association of active principles may be obtained by a step of mixing the xanthine oxidase inhibitor, febuxostat, or pharmaceutically acceptable salts thereof or polymorphic forms thereof with the angiotensin II receptor antagonist or pharmaceutically acceptable salts thereof, in a weight ratio comprised between 0.5 and 400, or between 2.5 and 120.

For the preparation of the pharmaceutical compositions described herein the mixture of active principles is formulated in suitable dosage units with one or more pharmaceutically acceptable excipients and additives.

### Testing

Testing demonstrating activity of the associations according to the invention is reported hereinafter.

### Biological activity

Pharmacological activity of febuxostat and olmesartan medoxomil, alone or in association, was assessed in an experimental model of hyperuricemia in spontaneously hypertensive rat (SHR, Harlan Laboratories, Udine, Italy).

Systolic pressure (SBP) was measured in non-anesthesized animals by means of a tail cuff sphygmomanometer (Blood Pressure Analysis System BP-2000, Visitech Systems, USA) equipped with an automatic system for data acquisition and processing.

All animals were preconditioned daily for 2 weeks to accustom them to instrumental measuring of the pressure before starting the test. Hyperuricemia was induced with administration of potassium oxonate (250 mg/kg IP, 2 hours after the last dose of drugs and 2 hours before the test), an uricase inhibitor capable of inducing experimental hyperuricemia. Plasma levels of uric acid were measured with a standard colorimetric enzymatic method. Febuxostat and/or olmesartan were administered orally once per day for 7 days, by gavage, at the doses of 1-3-10 mg/kg.

Parameters measured in conscious rats were systolic pressure (SBP) and plasma uric acid/urate levels (uricemia), as well as basal values, both after one week of treatment and 2 hours after administration of potassium oxonate. Blood was collected from a tail vein.

### Plasma uric acid

Basal values of plasma uric acid were comparable in all groups and comprised between 0.37±0.06 and 0.53±0.08 mg/dL. After one week of daily treatment with febuxostat and/or olmesartan, potassium oxonate administration produced a significant increase of plasma uric acid, higher than over 100% with respect to the basal value. Oral administration of febuxostat, for one week before the treatment with potassium oxonate, reduced in a significant and dose-dependent manner the hyperuricemia values (Table 1) whereas olmesartan medoxomil, *per se* weakly active, in combination with febuxostat has surprisingly increased the effect of febuxostat in reducing hyperuricemia to the dose of 3 mg/kg per os for each compound.

### Systolic pressure

Basal values of systolic pressure, measured with tail cuff sphygmomanometer in spontaneously hypertensive rats, were found homogeneous in the various groups and comprised between 219±4 and 228±3 mmHg. Potassium oxonate administration slightly increased systolic pressure, yet in a non-significant manner (Table 1).

Febuxostat (1-3-10 mg/kg per os, daily for 1 week) showed a slight inhibitory effect on systolic pressure. Olmesartan medoxomil (1-3-10 mg/kg per os, daily for a week) reduced systolic pressure in a dose-dependent manner (Table 1). Combined administration of febuxostat and olmesartan medoxomil, at the dose of 3 mg/kg per os, reduced the systolic pressure in spontaneously hypertensive rats to the values obtained with the dose of olmesartan medoxomil (10 mg/kg per os) showing consistent enhancement of the hypotensive effect of angiotensin II AT1 receptor.

**Table 1 - Effect of daily oral administration for one week of febuxostat, olmesartan medoxomil or combination thereof on uricemia and systolic pressure values in spontaneously hypertensive rats (SHR).**

| Treatment mg/kg day per os | Uricemia mg/dL | Δ Uricemia mg/dL | SBP mmHg | Δ SBP mmHg | |
|---|---|---|---|---|---|
| Control (vehicle) | - | 0.44±0.08 | - | 228±7 | - |
| | | | | | |
| Control+ K oxonate | - | 1.17±0.10 | - | 239±5 | - |
| | | | | | |
| Febuxostat | 1 | 1.02±0.07 | - 0.15 | 234±19 | -5 |
| | 3 | 0.78±0.06 | - 0.39 | 229±24 | -10 |
| | 10 | 0.49±0.05 | -0.68 | 228±21 | -11 |
| | | | | | |
| Olmesartan | 1 | 1.16±0.11 | - 0.01 | 217±14 | -22 |
| | 3 | 1.10±0.12 | - 0.07 | 202±10 | -37 |
| | 10 | 1.08±0.07 | - 0.09 | 180±11 | -59 |
| | | | | | |
| Febuxostat+Olmesartan 3+3 | | 0.59±0.05 | - 0.58 | 175±14 | -64 |

Uricemia and systolic pressure values were recorded 4 hours after the last dose of drugs or vehicle. Potassium oxonate (250 mg/kg IP) was administered 2 ore before the test. Each value is the average of 7-13 tests.

Comparable results of enhancement after combined administration were obtained also by assessing diastolic pressure.

Examples of pharmaceutical formulations according to the present invention are reported hereinafter.

### Example 1

| tablet for oral administration, containing: | |
|---|---|
| febuxostat | 120 mg |
| olmesartan medoxomil | 40 mg |
| pregelatinized starch (disintegrating binder) | 70 mg |
| silicified microcrystalline cellulose (filler) | 32.656 mg |
| croscarmellose sodium (disintegrant) | 10 mg |
| magnesium stearate (lubricant) | 0.8 mg |

### Example 2

| tablet for oral administration, containing: | |
|---|---|
| febuxostat | 80 mg |
| olmesartan medoxomil | 20 mg |
| pregelatinized starch (disintegrating binder) | 35 mg |
| silicified microcrystalline cellulose (filler) | 72.256 mg |
| croscarmellose sodium (disintegrant) | 5 mg |
| magnesium stearate (lubricant) | 0.4 mg |

### Example 3

| tablet for oral administration, containing: | |
|---|---|
| febuxostat | 40 mg |
| olmesartan medoxomil | 10 mg |
| pregelatinized starch (disintegrating binder) | 35 mg |
| silicified microcrystalline cellulose (filler) | 85.312 mg |
| croscarmellose sodium (disintegrant) | 5 mg |
| magnesium stearate (lubricant) | 0.4 mg |

## Claims

1. An association of the active principles:
a) xanthine oxidase inhibitor, febuxostat, or pharmaceutically acceptable salts thereof or polymorphic forms thereof; and
b) at least one angiotensin II receptor antagonists that is olmesartan medoxomil or pharmaceutically acceptable salts thereof
for use in a human or veterinary therapeutic treatment.

2. The association according to claim 1 wherein the active principle (a) is associated to the active principle (b) in a weight ratio of (a)/(b) comprised between 0.1 and 200.

3. The association according to claim 2, wherein said weight ratio is comprised between 0.6 and 10.

4. The association according to any one of claims 1 to 3, for use in the therapeutic treatment of hypertension.

5. The association according to any one of claims 1 to 4, for use in the therapeutic treatment of hypertension associated to hyperuricemia.

6. The association according to any one of claims 1 to 4, for use in the therapeutic treatment of hypertension associated to hyperuricemia and/or other disorders of the metabolic syndrome.

7. A pharmaceutical composition for use in a human or veterinary therapeutic treatment comprising, as active principle, a mixture of:
a) xanthine oxidase inhibitor, febuxostat, or pharmaceutically acceptable salts thereof or polymorphic forms thereof;
b) ay least one angiotensin II receptor antagonists, that is olmesartan medoxomil or pharmaceutically acceptable salts thereof;
and
one or more pharmaceutically acceptable excipients and/or additives.

8. The pharmaceutical composition according to claims 7, for use in the therapeutic treatment of hypertension.

9. The pharmaceutical composition according to any one of claims 7 to 8, for use in the therapeutic treatment of hypertension associated to hyperuricemia.

10. The pharmaceutical composition according to any one of claims 7 to 8, for use in the therapeutic treatment of hypertension associated to hyperuricemia and/or other disorders of the metabolic syndrome.

11. The pharmaceutical composition according to any one of claims 7 to 10, wherein the xanthine oxidase inhibitor febuxostat is in an amount comprised between 10 - 200 mg per dosage unit.

12. The pharmaceutical composition according to any one of claims 7 to 11, wherein the xanthine oxidase inhibitor febuxostat is in an amount comprised between 25 - 100 mg per dosage unit.

13. The pharmaceutical composition according to any one of claims 7 to 11, wherein the angiotensin II receptor antagonist olmesartan medoxomil is in an amount comprised between 1 - 100 mg per dosage unit.

14. The pharmaceutical composition according to any one of claims 7 to 11, wherein the angiotensin II receptor antagonist is in an amount comprised between 10 - 40 mg per dosage unit.

15. A method for the preparation of the composition according to any one of claims 7 to 14, wherein the active mixture comprising:
a) xanthine oxidase inhibitor, febuxostat, or pharmaceutically acceptable salts thereof or polymorphic forms thereof; and
b) at least one angiotensin II receptor antagonists, that is olmesartan medoxomil or pharmaceutically acceptable salts thereof
is formulated in suitable dosage units with one or more pharmaceutically acceptable excipients and/or additives.

## Patentansprüche

1. Kombination von Wirkstoffen:
a) Xanthinoxidaseinhibitor, Febuxostat oder pharmazeutisch verträgliche Salze davon oder polymorphe Formen davon; und
b) mindestens ein Angiotensin-II-Rezeptor-Antagonist, der Olmesartanmedoxomil oder pharmazeutisch verträgliche Salze davon ist,
zur Verwendung in einer menschlichen oder tierärztlichen therapeutischen Behandlung.

2. Kombination nach Anspruch 1, wobei der Wirkstoff (a) kombiniert ist mit dem Wirkstoff (b) in einem Gewichtsverhältnis von (a)/(b), umfassend zwischen 0,1 und 200.

3. Kombination nach Anspruch 2, wobei das Gewichtsverhältnis zwischen 0,6 und 10 umfasst.

4. Kombination nach einem der Ansprüche 1 bis 3, zur Verwendung in der therapeutischen Behandlung von Bluthochdruck.

5. Kombination nach einem der Ansprüche 1 bis 4, zur Verwendung in der therapeutischen Behandlung von Bluthochdruck, der Hyperurikämie begleitet.

6. Kombination nach einem der Ansprüche 1 bis 4, zur Verwendung in der therapeutischen Behandlung von Bluthochdruck, der Hyperurikämie und/oder andere Erkrankungen des metabolischen Syndroms begleitet.

7. Arzneimittel zur Verwendung in einer menschlichen oder tierärztlichen therapeutischen Behandlung, umfassend als Wirkstoff eine Mischung aus:
a) Xanthinoxidaseinhibitor, Febuxostat oder pharmazeutisch verträgliche Salze davon oder polymorphe Formen davon;
b) mindestens einen Angiotensin-II-Rezeptor-Antagonisten, der Olmesartanmedoxomil oder pharmazeutisch verträgliche Salze davon ist; und
eine oder mehrere pharmazeutisch verträgliche Exzipienten und/oder Additive.

8. Arzneimittel nach Anspruch 7 zur Verwendung in der therapeutischen Behandlung von Bluthochdruck.

9. Arzneimittel nach einem der Ansprüche 7 bis 8 zur Verwendung in der therapeutischen Behandlung von Bluthochdruck, der Hyperurikämie begleitet.

10. Arzneimittel nach einem der Ansprüche 7 bis 8 zur Verwendung in der therapeutischen Behandlung von Bluthochdruck, der Hyperurikämie und/oder andere Erkrankungen des metabolischen Syndroms begleitet.

11. Arzneimittel nach einem der Ansprüche 7 bis 10, wobei der Xanthinoxidaseinhibitor Febuxostat in einer Menge umfassend zwischen 10-200 mg pro Dosierungseinheit ist.

12. Arzneimittel nach einem der Ansprüche 7 bis 11, wobei der Xanthinoxidaseinhibitor Febuxostat in einer Menge umfassend zwischen 25-100 mg pro Dosierungseinheit ist.

13. Arzneimittel nach einem der Ansprüche 7 bis 11, wobei der Angiotensin-II-Rezeptor-Antagonist Olmesartanmedoxomil in einer Menge umfassend zwischen 1-100 mg pro Dosierungseinheit ist.

14. Arzneimittel nach einem der Ansprüche 7 bis 11, wobei der Angiotensin-II-Rezeptor-Antagonist in einer Menge umfassend zwischen 10-40 mg pro Dosierungseinheit ist.

15. Verfahren zur Herstellung einer Zusammensetzung nach einem der Ansprüche 7 bis 14, wobei der Wirkstoff umfasst:
a) Xanthinoxidaseinhibitor, Febuxostat oder pharmazeutisch verträgliche Salze davon oder polymorphe Formen davon; und
b) mindestens einen Angiotensin-II-Rezeptor-Antagonisten, der Olmesartanmedoxomil oder pharmazeutisch verträgliche Salze davon ist, formuliert in geeigneten Dosierungseinheiten mit einer oder mehreren pharmazeutisch verträglichen Exzipienten und/oder Additiven.

## Revendications

1. Association des principes actifs :
a) un inhibiteur de la xanthine oxydase, le fébuxostat, ou des sels pharmaceutiquement acceptables de celui-ci ou des formes polymorphes pharmaceutiquement acceptables de celui-ci ; et
b) au moins un antagoniste du récepteur de l'angiotensine II qui est l'olmésartan médoxomil ou des sels pharmaceutiquement acceptables de celui-ci pour une utilisation dans un traitement thérapeutique humain ou vétérinaire.

2. Association selon la revendication 1, dans laquelle le principe actif (a) est associé au principe actif (b) dans un rapport pondéral de (a)/(b) compris entre 0,1 et 200.

3. Association selon la revendication 2, dans laquelle ledit rapport pondéral est compris entre 0,6 et 10.

4. Association selon l'une quelconque des revendications 1 à 3, pour une utilisation dans le traitement thérapeutique de l'hypertension.

5. Association selon l'une quelconque des revendications 1 à 4, pour une utilisation dans le traitement thérapeutique de l'hypertension associée à l'hyperuricémie.

6. Association selon l'une quelconque des revendications 1 à 4, pour une utilisation dans le traitement thérapeutique de l'hypertension associée à l'hyperuricémie et/ou à d'autres troubles du syndrome métabolique.

7. Composition pharmaceutique pour une utilisation dans un traitement thérapeutique humain ou vétérinaire comprenant, en tant que principe actif, un mélange de :
a) un inhibiteur de la xanthine oxydase, le fébuxostat, ou des sels pharmaceutiquement acceptables de celui-ci ou des formes polymorphes pharmaceutiquement acceptables de celui-ci ;
b) au moins un antagoniste du récepteur de l'angiotensine II qui est l'olmésartan médoxomil ou des sels pharmaceutiquement acceptables de celui-ci ;
et
un ou plusieurs excipients et/ou additifs pharmaceutiquement acceptables.

8. Composition pharmaceutique selon la revendication 7, pour une utilisation dans le traitement thérapeutique de l'hypertension.

9. Composition pharmaceutique selon l'une quelconque des revendications 7 à 8, pour une utilisation dans le traitement thérapeutique de l'hypertension associée à l'hyperuricémie.

10. Composition pharmaceutique selon l'une quelconque des revendications 7 à 8, pour une utilisation dans le traitement thérapeutique de l'hypertension associée à l'hyperuricémie et/ou à d'autres troubles du syndrome métabolique.

11. Composition pharmaceutique selon l'une quelconque des revendications 7 à 10, dans laquelle l'inhibiteur de xanthine oxydase, le fébuxostat, est présent en une quantité comprise entre 10 et 200 mg par unité posologique.

12. Composition pharmaceutique selon l'une quelconque des revendications 7 à 11, dans laquelle l'inhibiteur de xanthine oxydase, le fébuxostat, est présent en une quantité comprise entre 25 et 100 mg par unité posologique.

13. Composition pharmaceutique selon l'une quelconque des revendications 7 à 11, dans laquelle l'antagoniste du récepteur d'angiotensine II, l'olmésartan médoxomil, est présent en une quantité comprise entre 1 et 100 mg par unité posologique.

14. Composition pharmaceutique selon l'une quelconque des revendications 7 à 11, dans laquelle l'antagoniste du récepteur d'angiotensine II est présent en une quantité comprise entre 10 et 40 mg par unité posologique.

15. Procédé de préparation de la composition selon l'une quelconque des revendications 7 à 14, dans lequel le mélange actif comprenant :
a) un inhibiteur de la xanthine oxydase, le fébuxostat, ou des sels pharmaceutiquement acceptables de celui-ci ou des formes polymorphes pharmaceutiquement acceptables de celui-ci ; et
b) au moins un antagoniste du récepteur de l'angiotensine II qui est l'olmésartan médoxomil ou des sels pharmaceutiquement acceptables de celui-ci est formulé dans des unités posologiques appropriées avec un ou plusieurs excipients et/ou additifs pharmaceutiquement acceptables.
